(19) Europäisches Patentamt / European Patent Office / Office européen des brevets

(11) **EP 3 796 321 A1**

(12) **EUROPEAN PATENT APPLICATION**
published in accordance with Art. 153(4) EPC

(43) Date of publication:
**24.03.2021 Bulletin 2021/12**

(51) Int Cl.:
*G16B 30/00* (2019.01)   *G16B 25/00* (2019.01)
*C12Q 1/6811* (2018.01)   *C12Q 1/6876* (2018.01)

(21) Application number: **19803857.2**

(22) Date of filing: **15.05.2019**

(86) International application number:
**PCT/KR2019/005809**

(87) International publication number:
**WO 2019/221498 (21.11.2019 Gazette 2019/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.05.2018 KR 20180055315**

(71) Applicant: PROGENEER INC.
**Guro-gu**
**Seoul 08380 (KR)**

(72) Inventors:
• **SHIN, Seung-Won**
  **Seoul 04747 (KR)**
• **UM, Soong-Ho**
  **Seoul 04354 (KR)**
• **LEE, Jung-Heon**
  **Seoul 06690 (KR)**
• **LEE, Byoung-Sang**
  **Suwon-si Gyeonggi-do 16362 (KR)**

(74) Representative: **Ladendorf, Oliver**
**Kraus & Weisert**
**Patentanwälte PartGmbB**
**Thomas-Wimmer-Ring 15**
**80539 München (DE)**

(54) **METHOD FOR DESIGNING ARTIFICIAL BASE SEQUENCE FOR BINDING TO POLYNUCLEIC ACID BIOMARKER, AND POLYNUCLEIC ACID PROBE USING SAME**

(57)    The present invention relates to a multiple-nucleic-acid probe comprising a single nucleotide sequence that binds to biomarkers for detection of multiple nucleic acids. An artificial nucleotide sequence designed by the method for designing an artificial nucleotide sequence according to the present invention exhibits better hybridization reactivity with all analogs, and a multiple-nucleic-acid probe comprising the artificial nucleotide sequence is designed such that a single diagnostic probe is capable of simultaneously binding to multiple types of nucleic acids having significance. Thus, use of the single diagnostic probe may achieve ultra-multiplex diagnosis of nucleic acid biomarkers by diagnosing the overall expression pattern in a sample, whereby diagnostic ability may be improved and the cost of examination may be drastically reduced.

【Fig. 2a】

**Description**

[Technical Field]

The present invention relates to designing a single nucleotide sequence that binds to biomarkers for detection of multiple nucleic acids, and a multiple-nucleic-acid probe comprising the same.

[Background Art]

Genetic testing can confirm or rule out a suspected genetic disease in patients having symptoms of the disease, and can also predict diseases that increase the likelihood and risk of developing diseases through testing for genetic mutations that cause diseases. It has been reported that such genetic testing can reduce disease morbidity and mortality by helping to prevent, diagnose early, treat and manage diseases.

Genetic testing can be performed on any tissue in the human body on the premise that all cells in the human body are genetically identical, but genetic testing can be most easily performed using nucleic acids extracted from blood.

The field of nucleic acid diagnosis has been used for determination of single nucleotide polymorphism (SNP), detection and identification of pathogenic bacteria or viruses, diagnosis of genetic diseases, and the like. Accordingly, a number of methods for quickly and accurately detecting specific nucleic acids have been proposed, and many studies related thereto are still underway (W. Shen et al., 2013, Biosen. and Bioele., 42:165-172.; M.L. Ermini et al., 2014, Biosen. and Bioele., 61:28-37.; K. Chang et al., 2015, Biosen. and Bioele., 66:297-307.). The most common methods that are used to detect specific nucleic acids comprise: methods that use polymerase chain reaction (PCR); multiplex polymerase chain reaction (multiplex PCR) methods; and SNPlex, GoldenGate assay, and molecular inversion probes (MIPs), which are techniques enabling high-throughput analysis by simultaneously amplifying multiple nucleic acids using common primers without using multiple polymerase chain reaction.

In recent years, a nucleic acid probe composed of a nucleotide sequence capable of complementarily binding to a target nucleic acid to be detected has been developed. The nucleic acid probe is a method of measuring biomarkers that hybridized with a biological sample, and a method of simultaneously detecting multiple biomarkers is essentially used in order to increase the significance of disease diagnosis and prognosis prediction. A conventional method is a method of performing multiple diagnoses using multiple nucleotide sequences capable of binding complementarily to single nucleic acid biomarkers.

However, the cost for multiple diagnoses is very high, and hence this conventional method has limitations in that it is difficult to use at an early stage and continuous monitoring and prognostic observation are difficult. For example, in the case of BRCA1 and BRCA2, which are representative breast cancer biomarkers, the probability of finding mutations in the actual patient group is low at about 13.9% (Journal of the Korean Society for Radiation Oncology), and in the case of commercially available diagnostic kits that are used for accurate diagnosis, it is possible to simultaneously diagnose an average of about 10 different genetic factors, but the cost per diagnostic test is $3,000 or more, and thus preventive diagnosis and continuous monitoring are severely limited.

Meanwhile, the k-means clustering algorithm belongs to the partitioning method among clustering methods. Partitioning is a method of dividing a given data set into several groups. For example, when it is assumed that n data objects are input, the partitioning method divides the input data into k groups, where k is smaller than or equal to the number (n) of the data objects n. Each of the divided groups forms clusters. That is, the data is divided into k groups, each consisting of one or more data objects. The process of dividing into groups is performed in a manner that minimizes a cost function such as dissimilarity between groups based on distance, and in this process, the similarity between data objects in the same group increases, and the similarity with the data objects in other groups decreases. The k-means algorithm determines the sum of squares of the distance between the centroid of each group and the data objects in the group as a cost function, and performs clustering by updating the group belonging to each data object in the direction that minimizes this function value.

The present inventors have made efforts to overcome the above-described disadvantages and to develop a method capable of quickly and inexpensively diagnosing the overall pattern of detectable nucleic acid biomarkers in a patient's biological sample, and as a result, have found that it is possible to develop a method of modeling an artificial nucleotide sequence having optimal selectivity for multiple nucleic acid biomarkers by using thermodynamic principles, comprising k-means clustering algorithms, and social network analysis, and it is possible to quickly and accurately detect multiple nucleic acids by the method, thereby completing the present invention.

[Disclosure]

[Technical Problem]

An object of the present invention is to provide a method for designing an artificial nucleotide sequence for binding to multiple nucleic acid biomarkers.

Another object of the present invention is to provide a multiple-nucleic-acid probe using the artificial nucleotide sequence.

Yet another object of the present invention is to provide a method of detecting multiple nucleic acids using the artificial nucleotide sequence.

[Technical Solution]

The present invention provides a method for designing an artificial nucleotide sequence for binding to multiple nucleic acid biomarkers, the method comprising steps of:

a) preparing each random analog sequence(standard sequence) set having similarity to target nucleic acids;

b) selecting two analog sequences having the highest hybridization profile similarity among the analog sequence set by using a nearest-neighbor algorithm;

c) setting multiple equilibrium reactions of a triple-stranded sequence consisting of the two selected analog sequences and an arbitrary nucleic acid sequence, and selecting a common complement, which indicates the sum of the highest equilibrium constants (K), by using the following Equation 1:

[Equation 1]

$$[\text{Analog A}] + [\text{Complement}] = [\text{Complex}_A] \cdots \Delta G_A = - RT\ln K_A$$

$$[\text{Analog B}] + [\text{Complement}] = [\text{Complex}_B] \cdots \Delta G_B = - RT\ln K_B$$

$$K_A = [\text{Complex}_A]/[\text{Analog A}][\text{Complement}] = x_A(4-x_A-x_B)/(1-x_A)(2-x_A-x_B)$$

$$K_B = [\text{Complex}_B]/[\text{Analog B}][\text{Complement}] = x_B(4-x_A-x_B)/(1-x_B)(2-x_A-x_B)$$

wherein [Analog] represents the concentration of the analog, [Complex] represents the concentration of a complex of the analog and the complement, the concentration of the sequence is assigned equal to the hybridization reactivity of the two analog sequences, $\Delta G$ represents the Gibbs free energy in the standard state, R represents the gas constant, T represents absolute temperature, and x represents concentration;

d) selecting, as a strand complementary to the common complement, a representative sequence which is representative of the two analog sequences; and

e) repeating steps a) to e) until a single strand remains.

The present invention also provides a probe for detecting multiple nucleic acid biomarkers, the probe comprising the artificial nucleotide sequence and a fluorescent substance.

[Advantageous effects]

An artificial nucleotide sequence designed by the method for designing an artificial nucleotide sequence according to the present invention exhibits better hybridization reactivity with all analogs, and a multiple-nucleic-acid probe comprising the artificial nucleotide sequence is designed such that a single diagnostic probe is capable of simultaneously binding to multiple types of nucleic acids having significance. Thus, the use of the single diagnostic probe may achieve ultra-multiplex diagnosis of nucleic acid biomarkers by diagnosing the overall expression pattern in a sample, whereby diagnostic ability may be improved and the cost of examination may be drastically reduced.

[Description of Drawings]

FIG. 1a is a view showing information on a random nucleotide sequence (standard sequence) obtained from CANADA 2.0 and mutant sequences thereof.

FIG. 1b shows the sociogram of the standard sequence and the mutant sequences. The standard sequence and the mutants are marked as nodes (blue circles), and the top 100 rated complementary sequences (white circles) having the highest Gibbs free energy are linked to their complements. The complementary sequences have two or more linkages marked as yellow circles.

FIG. 1c shows the number of shared complementary sequences (Closeness) in 1,000 sequences and mutants having the highest Gibbs free energy.

FIG. 2a is a flowchart for calculating a representative sequence from two analogs. A modified nearest-neighbor model was used in the verification process. Adjacent parameters that do not match in base pairs were assumed to have no effect on the Gibbs free energy, and the initial base pairs were also not considered in the calculation. The representative sequence was obtained from the highest common complement, and the concentration was adjusted to create an equal amount of hybridization between the analogs.

FIG. 2b is a hyperbolic graph obtained from a multiple-reaction equilibrium constant calculation. The hyperbolic graph approaches the (1,1) coordinates as the reaction constant (K) increases, and the cross-point of the two hyperbolas indicates the equilibrium state of the reaction.

FIG. 3a shows information on analog sequences used to calculate a representative sequence. The analog sequences had three differences, and the calculated representative sequence was hetero-sequenced for the two analogs.

FIG. 3b shows the multiple-reaction equilibrium of the CS and Anti-analogs.

FIG. 3c is a sociogram showing that the representative sequence shares more complementary candidates with the analogs.

FIG. 3d shows a comparison of the Pearson's correlation coefficients of the Gibbs free energy values against all of the complementary sequences between the analogs and the representative sequence.

FIG. 4a shows the hybridization efficiency of common complements (294346 and 281802) against the analogs.

FIG. 4b shows the hybridization efficiency of the anti-analogs and the common complements with analog concentration variation.

FIG. 4c shows the efficiency of hybridization when the two analogs existed together.

FIG. 4d shows the optimized representative sequences calculated from various concentrations of analogs.

FIG. 4e shows the Pearson's correlation coefficients of the optimized representative sequences against the analogs.

FIG. 5a shows sequence information on multiple analogs and the Gibbs energy and Pearson's correlation coefficient between the sequences.

FIG. 5b shows the Gibbs free energies of the calculated common complements and hybridization yields against the analogs.

FIG. 5c shows the average Pearson's correlation coefficient of the representative sequence for the standard sequence and the initial analogs using a representative sequence having 5 analogs and 3 mismatch bases.

FIG. 5d shows the hybridization yield and the ratio of the Pearson's correlation coefficients of various mutation base numbers.

[Best Mode]

The present invention relates to a multiple-nucleic-acid probe using a single nucleotide sequence that binds to biomarkers for detection of multiple nucleic acids, and a method for detecting multiple nucleic acids using the same.

Hereinafter, embodiments of the present invention will be described in detail with reference to the accompanying drawings so that those skilled in the art may easily implement the present invention. However, the present invention may be embodied in various different forms and is not limited to the embodiments described herein. In addition, in the drawings, parts not related to the description are omitted in order to clearly describe the present invention.

Throughout the present specification, it is to be understood that, when any part is referred to as "comprising" any component, it does not exclude other components, but may further comprise other components, unless otherwise specified.

As used throughout the present specification, terms of degree, such as "about" and "substantially", are used in the sense of "at, or nearly at, when given the manufacturing and material tolerances inherent in the stated circumstances", and are used to prevent any unconscientious violator from unduly taking advantage of the disclosure in which exact or absolute numerical values are given so as to help understand the invention. As used throughout the present specification, the term "step of (doing) ...." or "step of ...." does not mean "a step for ...".

[Mode for Invention]

First, the present invention provides a method for designing an artificial nucleotide sequence for binding to multiple nucleic acid biomarkers, the method comprising steps of:

a) preparing each random analog sequence set having similarity to target nucleic acids;
b) selecting two analog sequences having the highest hybridization profile similarity among the analog sequence set by using a nearest-neighbor algorithm;
c) setting multiple equilibrium reactions of a triple-stranded sequence consisting of the two selected analog sequences and an arbitrary nucleic acid sequence, and selecting a common complement, which indicates the sum of the highest equilibrium constants (K), by using the following Equation 1:

```
[Equation 1]
```

$$[\text{Analog A}] + [\text{Complement}] = [\text{Complex}_A] \cdots \Delta G_A = -RT\ln K_A$$

$$[\text{Analog B}] + [\text{Complement}] = [\text{Complex}_B] \cdots \Delta G_B = -RT\ln K_B$$

$$K_A = [\text{Complex}_A]/[\text{Analog A}][\text{Complement}] = x_A(4-x_A-x_B)/(1-x_A)(2-x_A-x_B)$$

$$K_B = [\text{Complex}_B]/[\text{Analog B}][\text{Complement}] = x_B(4-x_A-x_B)/(1-x_B)(2-x_A-x_B)$$

wherein [Analog] represents the concentration of the analog, [Complex] represents the concentration of a complex of the analog and the complement, the concentration of the sequence is assigned equal to the hybridization reactivity of the two analog sequences, $\Delta G$ represents the Gibbs free energy in the standard state, R represents gas constant, T represents absolute temperature, and x represents concentration;
d) selecting, as a strand complementary to the common complement, a representative sequence which is representative of the two analog sequences; and
e) repeating steps a) to e) until a single strand remains.

If the analog sequences have three or more multiple strands, the method may further comprise:

f) selecting a common complement, which indicates the sum of the highest equilibrium constants (K), through the following Equation 2 using the representative sequence of step d) and the concentration thereof:

[Equation 2]

$$[Representative] + [Common] = [Complex_R] \cdots \Delta G_R = -RTlnK_R$$

$$K_R = [Complex_R]/[Common][Representative] = x_R(2x-x_R)/(x-x_R)^2$$

wherein [Representative] represents the concentration of a strand complementary to the common complement, [Common] represents the concentration of the common complement, [Complex] represents the concentration of the common complement and the strand complementary thereto, $\Delta G$ represents the Gibbs free energy in the standard state, R represents the gas constant, T represents absolute temperature, and x represents concentration;

h) selecting, as a strand complementary to the common complement, a representative sequence which is representative of the two analog sequences; and

i) repeating steps a) to h) until a single strand remains.

The multiple strands preferably have 3 to 5 strands.

In the "step of preparing each random analog sequence set having similarity to target nucleic acids" may be a step of automatically producing a set of analog sequences complementary to target sequences by using a program.

The "target nucleic acid" refers to any nucleic acid of interest that may be present in a biological sample.

The "biomarker" is used to refer to a target molecule that indicates or is a sign of normal or abnormal process in an individual or a disease or other condition in an individual. More specifically, the "biomarker" is an anatomical, physiological, biochemical or molecular parameter associated with the presence of a specific physiological state or process, whether normal or abnormal, and if abnormal, whether chronic or acute.

When a biomarker indicates or is a sign of an abnormal process or a disease or other condition in an individual, that biomarker is generally described as being either over-expressed or under-expressed as compared to an expression level or value of the biomarker that indicates or is a sign of a normal process or an absence of a disease or other condition in an individual. "Up-regulation", "up-regulated", "over-expression", "over-expressed", and any variations thereof are used interchangeably to refer to a value or level of a biomarker in a biological sample that is greater than a value or level (or range of values or levels) of the biomarker that is typically detected in similar biological samples from healthy or normal individuals.

For verification of the representative sequence, Pearson's correlation coefficients of the Gibbs free energy values may be used, and preferably, the average Pearson's correlation coefficient of the representative sequence is higher than those of all the analogs constituting the analog sequence set of step a).

Through the verification, it can be confirmed that the representative sequence represents the sequences and hybridization profile of the two analogs.

The artificial nucleotide sequence obtained in the present invention may exhibit higher hybridization reactivity than all analogs constituting the analog sequence set in step a).

In step a), each analog sequence may consist of 8 to 10 bases.

In step c), two analog sequences with the highest closeness may be calculated first after the number of shared complementary sequences is expressed as closeness.

The nearest-neighbor algorithm that is used in step c) calculates a sequence having the highest similarity to the target sequences. Specifically, the nearest-neighbor algorithm calculates all pairwise "distances" between analogs in one individual and analogs in each of all individuals. For the algorithm, reference may be made to Bremner D, et al., (2005). "Output-sensitive algorithms for computing nearest-neighbor decision boundaries". Discrete and Computational Geometry 33 (4): 593604), which is known literature.

Although a variety of methods may be used to identify the network of arbitrary elements, the most intuitive method is a method of adjusting elements in a multidimensional space and then measuring the Euclidean distance therebetween. When this method is applied to nucleotides, it can be seen that, if dots representing two nucleotides are located close to each other, the nucleotides show high sequence similarity, and on the contrary, if the sequences of the nucleotides significantly differ from each other, the distance between the two dots will be longer. Therefore, adjusting nucleotides by spatial arrangement may be ideally used for network analysis.

However, because the sequences of nucleotides are diverse and the interactions thereof are complex, it is almost impossible to project the same onto a single coordinate system. Thus, basic social networking methodologies may help determine nucleotide similarity. In the present invention, similarity was determined by visualizing a sociogram.

The present invention also provides a probe for detecting multiple nucleic acid biomarkers, the probe comprising: a nucleotide sequence designed by the method; and a fluorescent substance.

The fluorescent substance is preferably one or more selected from the group consisting of cyanine fluorescent molecules, rhodamine fluorescent molecules, Alexa fluorescent molecules, FITC (fluorescein isothiocyanate) fluorescent molecules, FAM (5-carboxy fluorescein) fluorescent molecules, Texas Red fluorescent molecules, and fluorescein. More preferably, the fluorescent substance may be cyanine.

The present invention also provides a method for detecting multiple nucleic acids, the method comprising steps of:

a) collecting a sample containing target nucleic acids;
b) mixing the sample, a primer set having a nucleotide sequence complementary to the sample, a cleavage reagent and a probe produced according to claim 8, and then amplifying target nucleic acid-probe complexes by an extension reaction; and
d) measuring the amount of probe fragments isolated from the complexes amplified in step c).

The "sample" is used interchangeably herein to refer to any material, biological fluid, tissue, or cell obtained or otherwise derived from an individual. This comprises blood (comprising whole blood, leukocytes, peripheral blood mononuclear cells, buffy coat, plasma, and serum), sputum, tears, mucus, nasal washes, nasal aspirate, breath, urine, semen, saliva, peritoneal washing, cystic fluid, amniotic fluid, glandular fluid, lymph fluid, cytologic fluid, ascites, pleural fluid, nipple aspirate, bronchial aspirate, bronchial brushing, synovial fluid, joint aspirate, organ secretions, cells, a cell extract, and cerebrospinal fluid. This also comprises experimentally separated fractions of all of the preceding. For example, a blood sample may be fractionated into serum, plasma, or into fractions containing particular types of blood cells, such as red blood cells or white blood cells. If desired, a sample may be a combination of samples from an individual, such as a combination of a tissue and fluid sample. The term "sample" also comprises materials containing homogenized solid materials, such as from a stool sample, a tissue sample, or a tissue biopsy, for example. The term "sample" also comprises materials derived from a tissue culture or a cell culture. Any suitable methods for obtaining a biological sample can be employed; exemplary methods comprise, e.g., phlebotomy, swab (e.g., buccal swab), and a fine needle aspirate biopsy procedure. Exemplary tissues susceptible to fine needle aspiration comprise lymph node, lung, lung washes, BAL (bronchoalveolar lavage), thyroid, breast, pancreas, and liver. Samples may also be collected, e.g., by microdissection (e.g., laser capture microdissection (LCM) or laser microdissection (LMD)), bladder wash, smear (e.g., a PAP smear), or ductal lavage. A "biological sample" obtained or derived from an individual comprises a sample that has been processed in any suitable manner after being obtained from the individual.

Furthermore, a biological sample may be derived by taking biological samples from a number of individuals and pooling the same, or pooling an aliquot of each individual's biological sample. The pooled sample may be treated as a sample from a single individual.

Hereinafter, preferred examples are presented to aid in understanding the present invention. However, the following examples are provided for easier understanding of the present invention, and the contents of the present invention are not limited by the examples.

Preparation Example 1. Preparation of Experiments

All of the nucleotides used in the experiments were purchased from Integrated DNA Technologies, Inc. (Coralville, IA, USA). Initially, lyophilized nucleotides were dissolved in TE buffer (10 mM Tris, pH 8.0, 0.1 mM EDTA) to a concentration of 100 $\mu$M. The nucleotides were mixed at a ratio corresponding to each experiment with 100 mM NaCl for the hybridization process. The annealing process was performed in a Mastercycler Pro thermocycler from Eppendorf (Westbury, NY, USA). After heating at 95°C for 5 minutes, temperature was decreased gradually from 95°C to 25°C at a rate of 0.5°C per minute. The fluorescence intensities were measured using a SpectraMax M5 provided by Molecular Devices, Inc. (Sunnyvale, CA, USA).

Preparation Example 2. Calculation of Gibbs Free Energy, Pearson's Correlation Coefficient, Multiple Equilibrium Constant, and Closeness

All calculations were conducted using Python v2.7, comprising the Gibbs free energy, Pearson's correlation coefficient, multiple equilibrium constants, and closeness. NumPy v1.8.0rc1 and SciPy v0.13.0b1 were used for algorithmic efficiency. Details of the calculation methods and formulae are presented in supplementary information.

Preparation Example 3. Sociogram

Cytoscape v3.6.0 was used to visualize the sociogram. The standard sequence, the analogs, and the complementary candidates were used as nodes. The complementary candidates were connected to their relevant standard sequence or analogs. The position of nodes was determined through a prefuse force-directed layout.

Example 1. Synthesis of Artificial Nucleotide Sequences

1-1. Mapping Nucleotides according to Sequence Similarity

First, a model nucleotide sequence consisting of 10 random bases (standard sequence) was obtained from CANADA 2.0, analog sequences (mutants) were synthesized by changing the base of the standard sequence in a cumulative manner. Mut-1 was generated by a single base random mutation of the standard sequence, and the mutated base was transferred to the next mutant Mut-2. Thus, Mut-2 possessed two mismatched sequences from the standard sequence, one of Mut-1 and one of its own. In this way, a total of 10 mutants was generated. As the mutation number becomes higher (accumulation of mutations), the sequence difference between the standard sequence and the mutant increases.

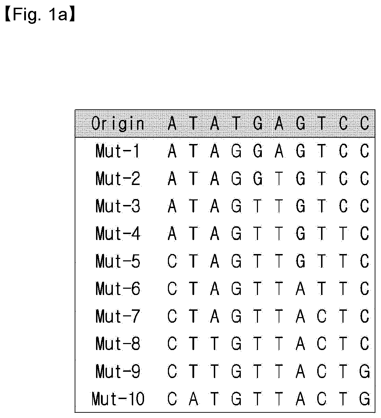

The sequence information on the standard sequence and mutants is shown in FIG. 1a, and the Gibbs free energy of all the possible complementary strands against the standard sequence and mutants was calculated.

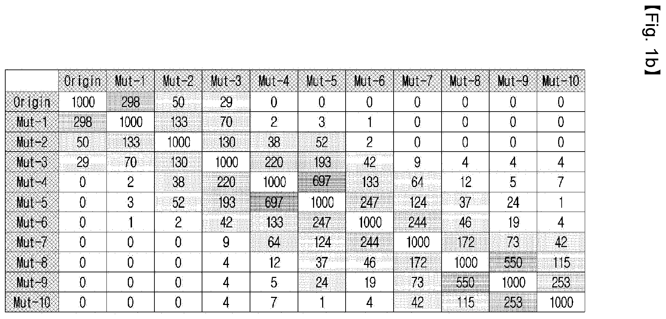

Since the sequence consisted of 10 bases, a total of $4^{10}$ complementary sequences was present. Among all possible complementary strands, 100 sequences with the highest Gibbs free energy were selected (complementary candidates), and connected to each standard sequence or mutant to draw a nondirectional sociogram as shown in FIG. 1B.

As shown in FIG. 1b, it was confirmed that, as the accumulation of mutations in the model nucleotide sequence increased, the number of shared complementary sequences decreased. The standard sequence shared most of the complementary sequences with one base mismatched nucleotide, Mut-1. In addition, the mismatched nucleotides shared most of the complementary sequences with their most similar analogs.

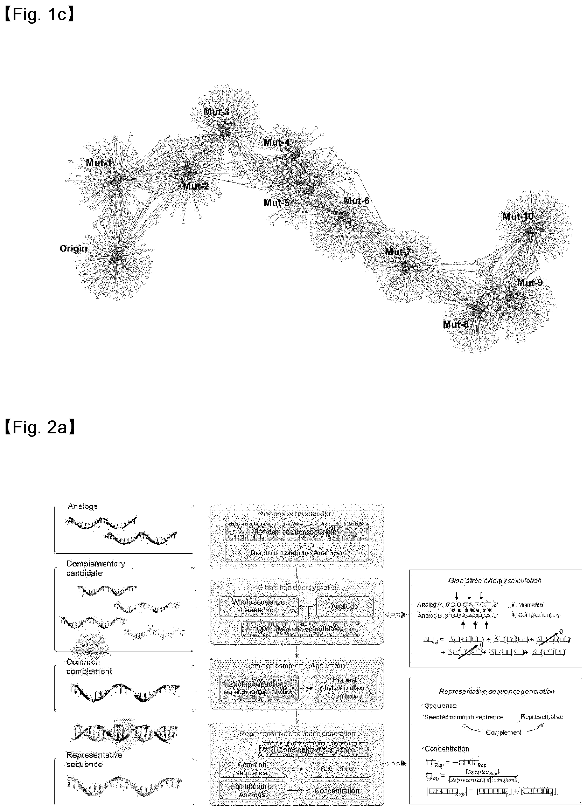

The number of shared complementary candidates was expressed as closeness, and the value of closeness was used to indicate similar Euclidean distances between nucleotides. The closeness profiles of the standard sequence and the mutants with the top 1000 rated complementary sequences are shown in FIG. 1c.

Cells contributing to the same sequence are indicated in yellow, and cells with a higher number of shared sequences are indicated in red. As shown in FIG. 1c, it was clear that all nucleotides had the highest closeness to the most similar bases. This indicates that it is possible to generate a representative sequence from multiple strands by mapping nucleotide sequences and mining a sequence located in the middle of the nucleotides.

1-2. Generation of Representative Sequence from Two Analogs

In the initial stage, two analogs were used as a model to prove the presence of the representative sequence. The calculation process is shown in FIG. 2a and is as follows.

1. The Gibbs free energy of all complementary strands for two analogs is calculated, and the complementary strands (complementary candidates) with the highest sum of Gibbs free energy are selected.

2. The equilibrium of multiple reactions of the complementary candidates and Gibbs free energy values is calculated, and the best complementary strand (common complement) with the highest reaction equilibrium sum is selected.

3. A representative sequence with a perfect antisense match to the common complement is generated, and the concentration of the representative sequence with an equal sum of the reaction equilibrium of the two analogs and the common complement is calculated.

First, in the procedure for the Gibbs free energy calculation, the nearest-neighbor model was used with some modifications. In general usage, the nearest-neighbor parameter of the nucleic acid duplex and the terminal base pair parameters should be comprised to calculate the enthalpy and entropy of hybridization. However, the present inventors considered only the nearest-neighbor parameter in the complementary base pairing. The nearest-neighbor parameters were referenced from a previous study. After calculation, the Gibbs free energy values of each complementary strand against two analogs were added, and 1000 strands with the highest Gibbs free energy were selected as the complementary candidates for the next equilibrium calculation step.

Since the Gibbs free energy values between the analog and the complementary candidates were calculated for a single reaction condition, the Gibbs free energy values should be converted to the reaction constants in multiple reactions, which contain both the analogs and the complementary candidate. The sum of the reaction constants of the two analogs indicated the involvement of the complementary candidate in hybridization. Then, the common complement with the highest sum of reaction constants was selected from the complementary candidates. The basic formula and calculation process are shown in Equation 1 below.

[Equation 1]

$$[\text{Analog A}] + [\text{Complement}] = [\text{Complex}_A] \quad \cdots \quad \Delta G_A = -RT\ln K_A$$

$$[\text{Analog B}] + [\text{Complement}] = [\text{Complex}_B] \quad \cdots \quad \Delta G_B = -RT\ln K_B$$

$$K_A = [\text{Complex}_A]/[\text{Analog A}][\text{Complement}] = x_A(4-x_A-x_B)/(1-x_A)(2-x_A-x_B)$$

$$K_B = [\text{Complex}_B]/[\text{Analog B}][\text{Complement}] = x_B(4-x_A-x_B)/(1-x_B)(2-x_A-x_B)$$

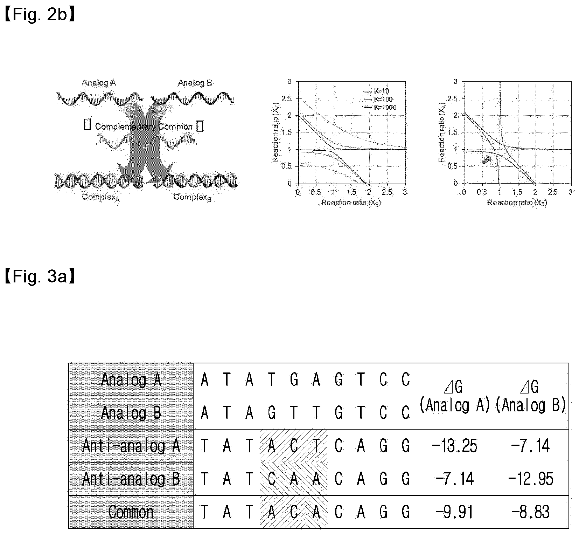

As shown in FIG. 2b, the formula of the reaction constant (K) was plotted in hyperbolic graphs, and the points of intersection in a reasonable range represent the multiple reaction equilibrium state (x). The representative sequence and the concentration thereof were calculated from the common complement. The representative sequence was designated as a perfect antisense sequence for the sequence of the common complement, and the concentration of the representative sequence was normalized by the sum of the reaction constants of the analog. The Gibbs free energy between the common complement and the representative sequence was also used in the calculation.

Two different sequences of nucleotides with two base mismatches were randomly selected for the analogs. The most favorable common complement and representative sequence were determined through calculations. Sequence information and hybridization Gibbs free energy of the analogs used to calculate the representative sequence are shown in FIG. 3a. The analogs had three differences in their sequences, and a calculated representative sequence was hetero-sequenced for both of the analogs.

As shown in FIG. 3a, the representative sequence was a hybrid form of the two analogs. As a result of comparing the Gibbs free energy values of the common complement and the perfect complement of the analogs, it was confirmed that the common complement did not have a maximized Gibbs free energy. However, the sum of the hybridization yield was higher than for the perfect complementary sequence.

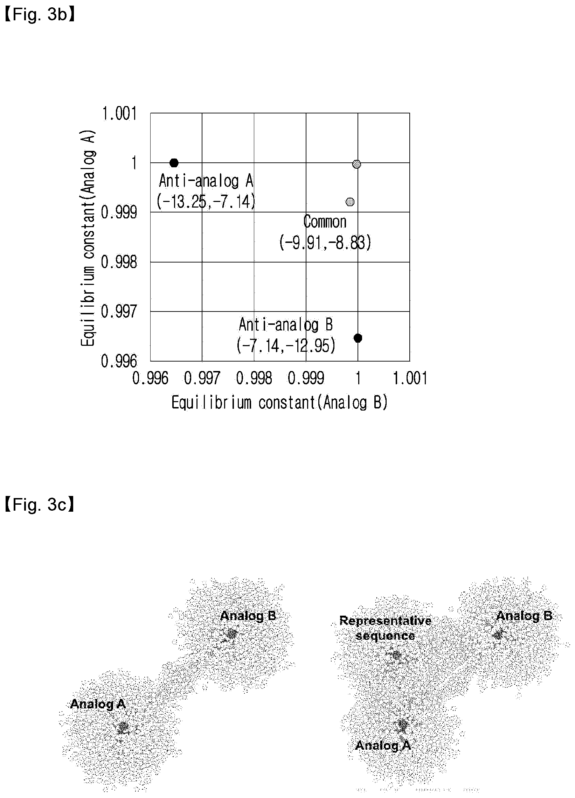

In addition, the multiple reaction equilibriums of the complement and the anti-analogs are shown in FIG. 3b.

As shown in FIG. 3b, the calculated multiple reaction equilibrium coordinates showed a closer distance to the (1,1) coordinates than the coordinates of the perfect complementary sequence.

In addition, FIG. 3c is a sociogram showing the complementary candidates shared between the representative sequence and the analogs.

As shown in FIG. 3c, it was confirmed that the representative sequence shared more complementary candidates with the analogs.

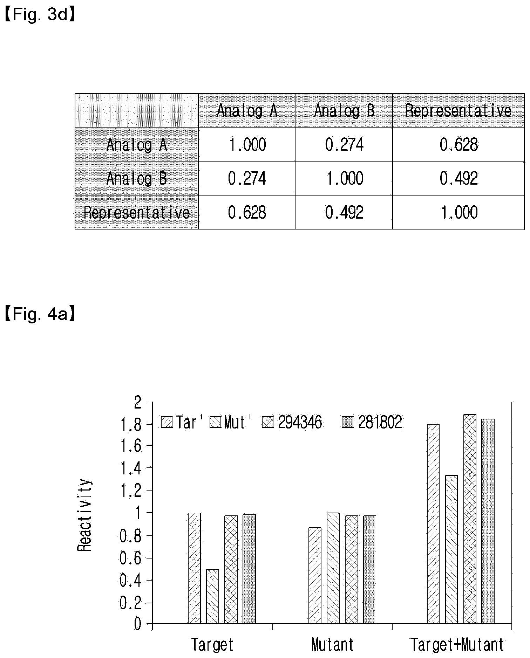

In addition, FIG. 3d shows a comparison of the Pearson's correlation coefficients of the Gibbs free energy values against all of the complementary sequences between the analogs and the representative sequence.

As shown in FIG. 3d, the Pearson's correlation coefficient of the Gibbs free energy values was used to show the similarity of the hybridization profile, and as expected, it was confirmed that the representative sequence had a higher average Pearson's correlation than the analogs.

From the above results, it was confirmed that the representative sequence can represent the sequences and hybridization profile of the two analogs.

1-3. Generation of Representative Sequence from Multiple Sequences

In the coordinate system, the K-means clustering algorithm can generate intuitive and rational centroids for clustering. For clustering with the K-means algorithm, the sum of distances from the centroid to the data objects is measured, and the coordinates of centroids are updated to minimize the sum. The centroid itself has a coordinate just like other data objects, although it is not real. Thus, it can be said that the centroid represents the properties of the data objects in the cluster. This is quite similar to the calculation of the representative sequence from the analog sequences. The present inventors tried to apply the K-means clustering algorithm to the generation of the representative sequence with multiple sequences.

In the existing k-means clustering algorithm, the distance from the centroid is readjusted to the centroid of the data objects in clustering to minimize the sum of distances. However, it is almost impossible to calculate the centroids of nucleotides with a large number of bases. Several remarkable approaches have been developed to calculate multiple nucleotide equilibrium states. For instance, Robert Dirt and his colleagues developed methodologies for calculating multi-strand interactions and the formation of secondary structures by the combination of graph theory and a partition function. However, tremendous resources and calculation times are needed to obtain reasonable results for thousands of reactions simultaneously. Therefore, the present inventors applied the k-means clustering algorithm in a step-by-step manner.

The difference arising in the stepwise calculation of the k-means clustering algorithm can be overcome by assigning mass to the data. For example, as indicated below, the centroid coordinates (x, y) of three points (a, b, c) in a two-dimensional space are the average of the coordinates.

$$(x, y) = ((x_a+x_b+x_c)/3, (y_a+y_b+y_c)/3)$$

$$(x, y)_{ab} = ((x_a+x_b)/2, (y_a+y_b)/2)$$

$$(x, y) = ([\{(x_a+x_b)/2\}+x_c]/2, [\{(y_a+y_b)/2\}+y_c]/2)$$

Meanwhile, when certain masses ($\alpha, \beta, \gamma$) are comprised, the centroid coordinates are expressed as follows.

$$(x, y) = ((\alpha x_a+\beta x_b+\gamma x_c)/(\alpha+\beta+\gamma), (\alpha y_a+\beta y_b+\gamma y_c)/(\alpha+\beta+\gamma))$$

In this case, stepwise calculation of the centroid coordinates is as follows, and the coordinate centroid end will be the same as that of a single calculation.

Example 2. Experimental Verification of Synthesized Nucleotide Sequence

To experimentally demonstrate multiple reactions and hybridization between the analogs and the common complement, two common complements (code numbers: 294346 and 281802) were selected, and the efficiencies of hybridization of the common complements to the analogs are shown in FIG. 4a. For measurement, analogs were labeled with fluorescent dyes (Cy3 and Cy5), and common complements were labeled with Iowa Black quencher. When hybridization between the analogs and the common complements occurs, the fluorescence intensities become weaker.

As shown in FIG. 4a, first, 1 µM of each analog (Analog 1 or Analog 2) was combined with 2 µM of its perfect complementary sequences (anti-analogs) separately. As expected, the anti-analogs showed the highest hybridization efficiency with their own analog. However, hybridization to the other analog was not effective. In the case of anti-Analog 2, perfect hybridization was shown with Analog 2. Meanwhile, anti-Analog 2 hybridized to Analog 1 with only 50.4% efficiency. Even though the common complements showed a lower hybridization efficiency than the perfect anti-analogs, hybridization with both analogs was better. Moreover, in the solution with the mixed Analogs, the common complements (code numbers: 294346 and 281802) showed the highest yield in total hybridization.

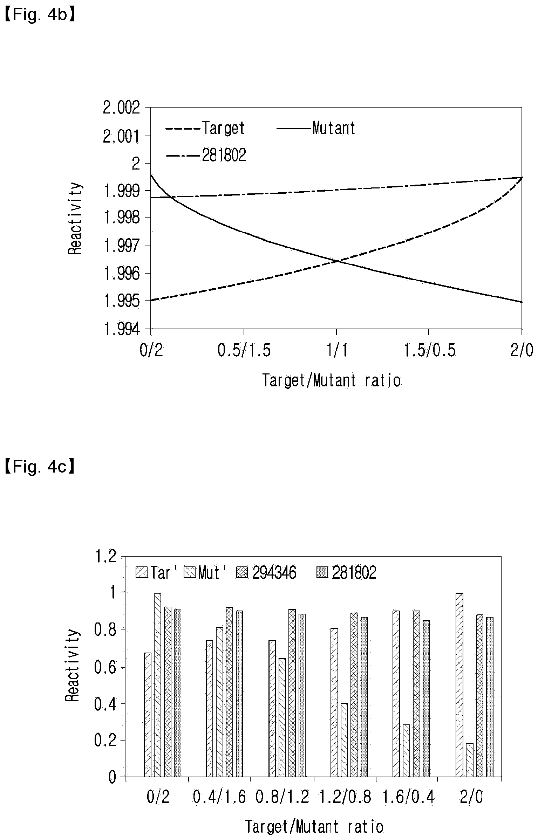

This phenomenon was also observed when the concentration of the analogs increased. Before the actual experiment, the hybridization efficiency of the Anti-analogs and the common complements in various concentrations of analogs was calculated, and the results of the calculation are shown in FIG. 4b.

As shown in FIG. 4b, the concentrations of the analogs were increased from 0 µM to 2 µM, and the sum of the concentrations was fixed at 2 µM. At the end-points and nearby, where Analog 1 or Analog 2 occupied all of the nucleotides at 2 µM, the anti-analogs showed the highest hybridization efficiencies. However, hybridization of the anti-analogs significantly decreased with the decrement of their own complements. In contrast, the common complements demonstrated sustained hybridization efficiency at all concentrations.

In addition, FIG. 4c shows the hybridization efficiency with the common complement when two analogs are present together.

As shown in FIG. 4c, when the two analogs were present together, the common complement showed higher hybridization efficiency. In addition, better hybridization efficiency was observed in the middle region with respect to the proportion of the analogs. The triangle region where the common complements showed higher hybridization efficiency in FIG. 4b well-described the potential of the common complements and the representative sequences. This tendency was also observed in the actual experiments, and this was also observed in FIG. 4c. The hybridization efficiency of the anti-analogs became lower with the increment of their less-compatible targets, but the common complements showed better hybridization at an analog 1/analog 2 ratio of 0.4:1.6 to 1.2:0.8, thus demonstrating the possibility of the representative sequences.

To make this result more reliable, 100 random analog sets were used to generate the representative sequences. As a result, even though there were differences in the hybridization efficiency values, the results showed solid evidence of the same process in the representative sequences. The Gibbs free energy profile of the analogs and the representative sequences against all possible complementary sequences were compared using Pearson's correlation coefficient. Between two-base mismatched nucleotides, the Pearson's correlation coefficient was 0.636 ± 0.049. In contrast, the average Pearson's correlation coefficient between the representative sequence and the analogs was 0.805 ± 0.042. This increment of the coefficient indicated that the representative sequence can delegate the hybridization profile of the analog sequences.

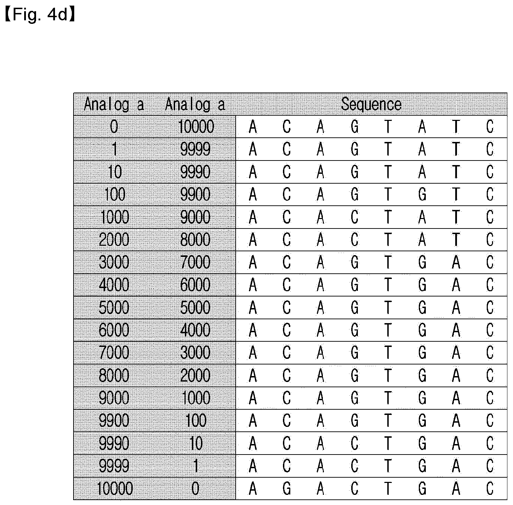

In addition, it was possible to calculate the optimized representative sequences from various concentrations of analogs, and the results of the calculation are shown in FIG. 4d.

As shown in FIG. 4d, two analogs sharing five of a total of eight bases were used to calculate the representative sequence in various concentrations. Analog concentrations were applied from 0:10,000 to 10,000:0, and optimized representative sequences were obtained.

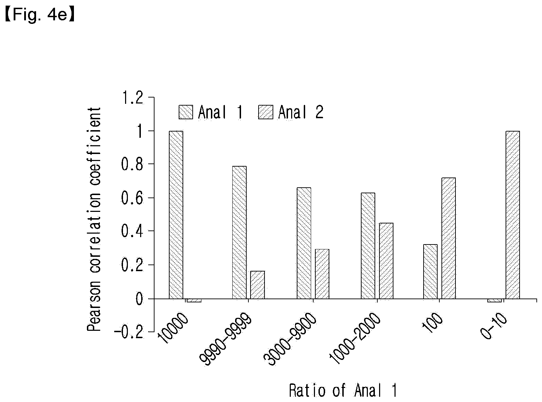

In addition, Pearson's correlation coefficients of the optimized representative sequences for the analogs were calculated, and the results of the calculation are shown in FIG. 4e.

As shown in FIG. 4e, with the concentration biases, the optimized representative sequences had greater closeness and a higher Pearson's correlation coefficient than the dominant analog.

Example 3. Theoretical verification of Synthesized Nucleotide Sequence

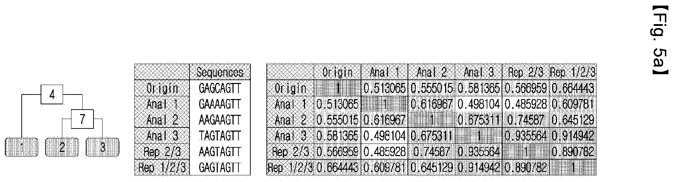

In order to verify the representative sequence, the sequence information of several analogs, Gibbs energy and Pearson's correlation coefficient between the sequences were calculated, and the results are shown in FIG. 5a.

As shown in FIG. 5a, three analogs were generated from a single nucleotide sequence having two mutations. As can be seen from the analysis of the two analogs, the Gibbs free energy value of analog 2/3 was lower than that of the perfect antisense sequence, but higher than that of the anti-analogs. The Pearson's correlation coefficient of the initial analogs to the standard sequence was 0.550, and the average coefficient therebetween was 0.597. The Pearson's correlation coefficients of Analog 2/3 against Analog 2 and Analog 3 (0.746 and 0.936, respectively) were higher than the coefficient between Analog 2 and Analog 3 (0.675). This result indicates that analog 2/3 is representative of analogs 2 and 3.

In contrast, the Pearson's correlation coefficient of Analog 2/3 against Analog 1 did not increase, and there was no increase in the Pearson's correlation coefficient to the standard sequence. This indicates that the calculated representative sequence was specific to the target analogs. Meanwhile, the representative sequence calculated from Analog 1 and Analog 2/3 showed an increment of the Pearson's correlation coefficient against the standard sequence and modest Gibbs free energy values. The coefficient value was 0.664, which was higher than the coefficient value of any other analog. The average coefficient was increased within all the analogs, and the average Pearson's correlation coefficient of the initial analogs against the representative sequence was higher than that against the standard sequence. This result not only demonstrates that it was possible to make a representative sequence, but also that its performance might be better than that of the standard sequence.

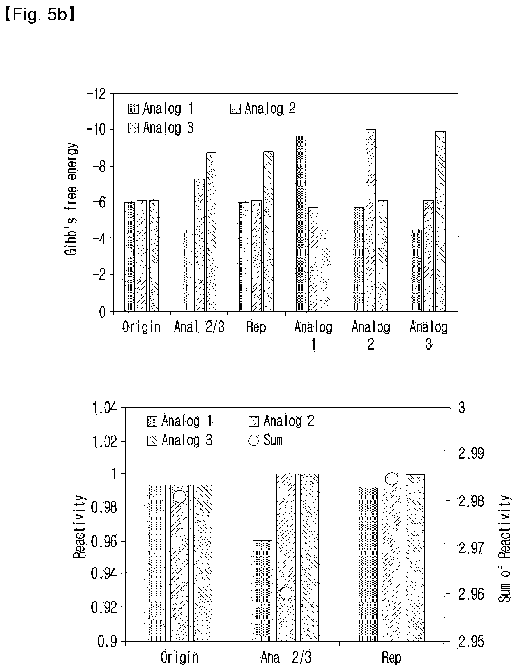

The potential of the representative sequence was also revealed in the equilibrium constant calculation, and the results of the calculation are shown in FIG. 5b.

As shown in FIG. 5b, the perfect complementary sequences of the standard sequence, Analog 2/3, and the representative sequence were used to measure the hybridization efficiency. The equilibrium constant for hybridization was calculated by the multiple-reaction equilibrium equation. The equilibrium constants of the antisense standard sequence were similar in the three analogs, and the sum of the constants was 2.980. In the case of anti-analog 2/3, which was calculated from analog 2 and analog 3, the equilibrium constants of analog 2 and analog 3 increased compared to the anti-analogs. However, the constant of analog 1 significantly decreased. Thus, the sum of the constants decreased overall (2,960). In addition, the antisense representative sequence showed a recovered hybridization yield of analog 1, and the sum of the constant was the highest (2,984). Through this result, the potential of the generated representative sequence was proved directly.

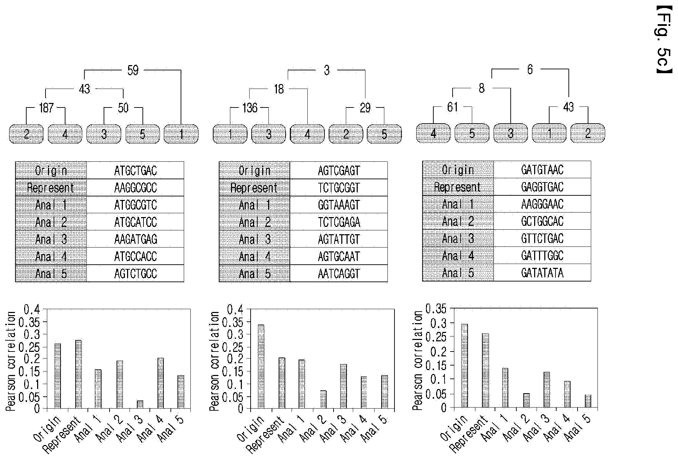

In addition, for analysis of multiple nucleotides, the representative sequences were obtained by the same procedure with five analogs and three mismatched bases. The average Pearson's correlations of the standard sequence and the representative sequence against the initial analogs were calculated, and the results of the calculation are shown in FIG. 5c.

As shown in FIG. 5c, the Pearson's correlation coefficient of the representative sequence was higher or lower than the Pearson's correlation of the standard sequence in a sequence-dependent manner; however, generally, it was higher than the coefficients of the analogs.

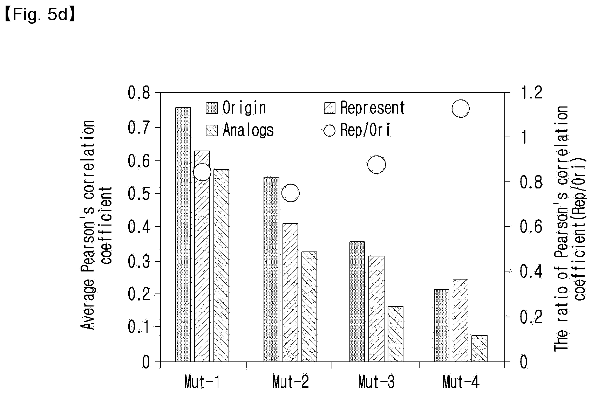

In addition, FIG. 5d shows the hybridization yield and the ratio of the Pearson's correlation coefficients of various mutation bases.

As shown in FIG. 5d, the average Pearson's correlation coefficient of the standard sequence against the analogs decreased with the increment of the number of mutations because the difference of the sequences led to less similarity in the hybridization profile. The decrement of the Pearson's correlation coefficient was also observed in the representative sequence. However, the amount of decrease was smaller than that of the standard sequence. Thus, the ratio of the Pearson's correlation coefficient (RS/origin) was increased with the number of mutations. When the number of mutations was 4, the average coefficient of the representative sequence was higher than that of the standard sequence. This result indicated that the procedure of the present invention can generate representative sequences from multiple nucleotides; however, they are not optimized as in the standard sequence. The present inventors believe that this shortage was generated from the imprecise calculation of the Gibbs free energy and the equilibrium constant. Especially, since there are several factors to consider in nucleotide hybridization, such as secondary structure, the equilibrium constant calculation with simple thermodynamic principles may not be sufficient for specific optimization for representative sequence generation. To overcome these inaccuracies, more complex and simultaneous calculations could be applied in the Gibbs energy and equilibrium state calculation process. However, it was confirmed that the representative sequence showed a much higher correlation with the analogs than any of the single analogs.

## Claims

1. A method for designing an artificial nucleotide sequence for binding to multiple nucleic acid biomarkers, the method comprising steps of:

    a) preparing each random analog sequence set having similarity to target nucleic acids;
    b) selecting two analog sequences having the highest hybridization profile similarity among the analog sequence set by using a nearest-neighbor algorithm;
    c) setting multiple equilibrium reactions of a triple-stranded sequence consisting of the two selected analog sequences and an arbitrary nucleic acid sequence, and selecting, as a common complement, the arbitrary nucleic acid sequence which indicates the sum of the highest equilibrium constants (K); and
    d) selecting, as a strand complementary to the common complement, a representative sequence which is representative of the two analog sequences.

2. The method of claim 1, wherein the average Pearson's correlation coefficient of the representative sequence is higher than those of all analogs constituting the analog sequence set in step a).

3. The method of claim 1, wherein the artificial nucleotide sequence exhibits higher hybridization reactivity than all analogs constituting the analog sequence set in step a) .

4. The method of claim 1, wherein each of the analog sequences consists of 8 to 10 bases.

5. The method of claim 1, wherein, in step c), two analog sequences with the highest closeness are calculated first after the number of shared complementary sequences is expressed as closeness.

6. A probe detecting multiple nucleic acid biomarkers, the probe comprising: a nucleotide sequence designed according to the method of claim 1; and a fluorescent substance.

7. The probe of claim 6, wherein the fluorescent substance is one or more selected from the group consisting of cyanine fluorescent molecules, rhodamine fluorescent molecules, Alexa fluorescent molecules, FITC (fluorescein isothiocyanate) fluorescent molecules, FAM (5-carboxy fluorescein) fluorescent molecules, Texas Red fluorescent molecules, and fluorescein.

【Fig. 1a】

| Origin | A | T | A | T | G | A | G | T | C | C |
|--------|---|---|---|---|---|---|---|---|---|---|
| Mut-1 | A | T | A | G | G | A | G | T | C | C |
| Mut-2 | A | T | A | G | G | T | G | T | C | C |
| Mut-3 | A | T | A | G | T | T | G | T | C | C |
| Mut-4 | A | T | A | G | T | T | G | T | T | C |
| Mut-5 | C | T | A | G | T | T | G | T | T | C |
| Mut-6 | C | T | A | G | T | T | A | T | T | C |
| Mut-7 | C | T | A | G | T | T | A | C | T | C |
| Mut-8 | C | T | T | G | T | T | A | C | T | C |
| Mut-9 | C | T | T | G | T | T | A | C | T | G |
| Mut-10 | C | A | T | G | T | T | A | C | T | G |

[Fig. 1b]

|  | Origin | Mut-1 | Mut-2 | Mut-3 | Mut-4 | Mut-5 | Mut-6 | Mut-7 | Mut-8 | Mut-9 | Mut-10 |
|---|---|---|---|---|---|---|---|---|---|---|---|
| Origin | 1000 | 298 | 50 | 29 | 0 | 0 | 0 | 0 | 0 | 0 | 0 |
| Mut-1 | 298 | 1000 | 133 | 70 | 2 | 3 | 1 | 0 | 0 | 0 | 0 |
| Mut-2 | 50 | 133 | 1000 | 130 | 38 | 52 | 2 | 0 | 0 | 0 | 0 |
| Mut-3 | 29 | 70 | 130 | 1000 | 220 | 193 | 42 | 9 | 4 | 4 | 4 |
| Mut-4 | 0 | 2 | 38 | 220 | 1000 | 697 | 133 | 64 | 12 | 5 | 7 |
| Mut-5 | 0 | 3 | 52 | 193 | 697 | 1000 | 247 | 124 | 37 | 24 | 1 |
| Mut-6 | 0 | 1 | 2 | 42 | 133 | 247 | 1000 | 244 | 46 | 19 | 4 |
| Mut-7 | 0 | 0 | 0 | 9 | 64 | 124 | 244 | 1000 | 172 | 73 | 42 |
| Mut-8 | 0 | 0 | 0 | 4 | 12 | 37 | 46 | 172 | 1000 | 550 | 115 |
| Mut-9 | 0 | 0 | 0 | 4 | 5 | 24 | 19 | 73 | 550 | 1000 | 253 |
| Mut-10 | 0 | 0 | 0 | 4 | 7 | 1 | 4 | 42 | 115 | 253 | 1000 |

【Fig. 1c】

【Fig. 2a】

【Fig. 2b】

【Fig. 3a】

| | | | | | | | | | | | $\Delta G$ | $\Delta G$ |
|---|---|---|---|---|---|---|---|---|---|---|---|---|
| Analog A | A | T | A | T | G | A | G | T | C | C | (Analog A) | (Analog B) |
| Analog B | A | T | A | G | T | T | G | T | C | C | | |
| Anti-analog A | T | A | T | A | C | T | C | A | G | G | -13.25 | -7.14 |
| Anti-analog B | T | A | T | C | A | A | C | A | G | G | -7.14 | -12.95 |
| Common | T | A | T | A | C | A | C | A | G | G | -9.91 | -8.83 |

【Fig. 3b】

【Fig. 3c】

【Fig. 3d】

|  | Analog A | Analog B | Representative |
|---|---|---|---|
| Analog A | 1.000 | 0.274 | 0.628 |
| Analog B | 0.274 | 1.000 | 0.492 |
| Representative | 0.628 | 0.492 | 1.000 |

【Fig. 4a】

【Fig. 4b】

【Fig. 4c】

【Fig. 4d】

| Analog a | Analog a | Sequence | | | | | | | |
|---|---|---|---|---|---|---|---|---|---|
| 0 | 10000 | A | C | A | G | T | A | T | C |
| 1 | 9999 | A | C | A | G | T | A | T | C |
| 10 | 9990 | A | C | A | G | T | A | T | C |
| 100 | 9900 | A | C | A | G | T | G | T | C |
| 1000 | 9000 | A | C | A | C | T | A | T | C |
| 2000 | 8000 | A | C | A | C | T | A | T | C |
| 3000 | 7000 | A | C | A | G | T | G | A | C |
| 4000 | 6000 | A | C | A | G | T | G | A | C |
| 5000 | 5000 | A | C | A | G | T | G | A | C |
| 6000 | 4000 | A | C | A | G | T | G | A | C |
| 7000 | 3000 | A | C | A | G | T | G | A | C |
| 8000 | 2000 | A | C | A | G | T | G | A | C |
| 9000 | 1000 | A | C | A | G | T | G | A | C |
| 9900 | 100 | A | C | A | G | T | G | A | C |
| 9990 | 10 | A | C | A | C | T | G | A | C |
| 9999 | 1 | A | C | A | C | T | G | A | C |
| 10000 | 0 | A | G | A | C | T | G | A | C |

【Fig. 4e】

【Fig. 5a】

| Sequences | |
|---|---|
| Origin | GAGCAGTT |
| Anal 1 | GAAAAGTT |
| Anal 2 | AAGAAGTT |
| Anal 3 | TAGTAGTT |
| Rep 2/3 | AAGTAGTT |
| Rep 1/2/3 | GAGTAGTT |

| | Origin | Anal 1 | Anal 2 | Anal 3 | Rep 2/3 | Rep 1/2/3 |
|---|---|---|---|---|---|---|
| Origin | 1 | 0.513065 | 0.555015 | 0.581365 | 0.566959 | 0.664443 |
| Anal 1 | 0.513065 | 1 | 0.616967 | 0.498104 | 0.485928 | 0.609781 |
| Anal 2 | 0.555015 | 0.616967 | 1 | 0.675311 | 0.74587 | 0.645129 |
| Anal 3 | 0.581365 | 0.498104 | 0.675311 | 1 | 0.935564 | 0.914942 |
| Rep 2/3 | 0.566959 | 0.485928 | 0.74587 | 0.935564 | 1 | 0.890782 |
| Rep 1/2/3 | 0.664443 | 0.609781 | 0.645129 | 0.914942 | 0.890782 | 1 |

【Fig. 5b】

【Fig. 5c】

【Fig. 5d】

<div style="text-align:center">

**INTERNATIONAL SEARCH REPORT**

</div>

| | International application No. |
|---|---|
| | **PCT/KR2019/005809** |

| A. CLASSIFICATION OF SUBJECT MATTER |
|---|
| *G16B 30/00(2019.01)i, G16B 25/00(2019.01)i, C12Q 1/6811(2018.01)i, C12Q 1/6876(2018.01)i* |
| According to International Patent Classification (IPC) or to both national classification and IPC |

| B. FIELDS SEARCHED |
|---|
| Minimum documentation searched (classification system followed by classification symbols) |
| G16B 30/00; C06F 7/00; C12Q 1/68; G01N 33/48; G01N 33/50; G06F 19/20; G16B 25/00; C12Q 1/6811; C12Q 1/6876 |
| Documentation searched other than minimum documentation to the extent that such documents are included in the fields searched<br>Korean utility models and applications for utility models: IPC as above<br>Japanese utility models and applications for utility models: IPC as above |
| Electronic data base consulted during the international search (name of data base and, where practicable, search terms used)<br>eKOMPASS (KIPO internal) & Keywords: artificial base sequence, multiple nucleic acid biomarker, k-means clustering algorithm, multiple equilibria, hybridization profile |

| C. DOCUMENTS CONSIDERED TO BE RELEVANT |
|---|

| Category* | Citation of document, with indication, where appropriate, of the relevant passages | Relevant to claim No. |
|---|---|---|
| A | US 2003-0077607 A1 (HOPFINGER, Anton J. et al.) 24 April 2003<br>See abstract; paragraph [0235]; and claims 4, 5, 14. | 1-7 |
| A | DE GUIRE, Vincent et al. Designing small multiple-target artificial RNAs. Nucleic Acids Research. 2010, vol. 38, no. 13, e140, pages 1-8<br>See the entire document. | 1-7 |
| A | TANAKA, Fumiaki et al. Design of nucleic acid sequences for DNA computing based on a thermodynamic approach. Nucleic Acids Research. 2005, vol. 33, no. 3, pages 903-911<br>See the entire document. | 1-7 |
| A | US 7117095 B2 (HUBBELL, Earl) 03 October 2006<br>See the entire document. | 1-7 |
| A | WO 2017-109762 A1 (DIASORIN S.P.A.) 29 June 2017<br>See the entire document. | 1-7 |

| ☒ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 20 AUGUST 2019 (20.08.2019) | **20 AUGUST 2019 (20.08.2019)** |
| Name and mailing address of the ISA/KR<br>Korean Intellectual Property Office<br>Government Complex Daejeon Building 4, 189, Cheongsa-ro, Seo-gu,<br>Daejeon, 35208, Republic of Korea<br>Facsimile No. +82-42-481-8578 | Authorized officer<br><br>Telephone No. |

Form PCT/ISA/210 (second sheet) (January 2015)

**INTERNATIONAL SEARCH REPORT**

| International application No. |
| --- |
| PCT/KR2019/005809 |

| C (Continuation). | DOCUMENTS CONSIDERED TO BE RELEVANT | |
| --- | --- | --- |
| Category* | Citation of document, with indication, where appropriate, of the relevant passages | Relevant to claim No. |
| PX | LEE, Byoungsang et al. Revealing the Presence of a Symbolic Sequence Representing Multiple Nucleotides Based on K-Means Clustering of Oligonucleotides. Molecules. 18 January 2019, vol. 24, Article No. 348, pages 1-13<br>See pages 3-10; and figure 2. | 1-7 |

Form PCT/ISA/210 (continuation of second sheet) (January 2015)

INTERNATIONAL SEARCH REPORT
Information on patent family members

International application No.

**PCT/KR2019/005809**

| Patent document cited in search report | Publication date | Patent family member | Publication date |
|---|---|---|---|
| US 2003-0077607 A1 | 24/04/2003 | AU 2002-252297 A1 | 24/09/2002 |
| | | WO 02-072868 A2 | 19/09/2002 |
| | | WO 02-072868 A3 | 16/10/2003 |
| US 7117095 B2 | 03/10/2006 | US 2002-0133301 A1 | 19/09/2002 |
| WO 2017-109762 A1 | 29/06/2017 | EP 3394777 A1 | 31/10/2018 |
| | | US 2018-0349553 A1 | 06/12/2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **W. SHEN et al.** *Biosen. and Bioele.,* 2013, vol. 42, 165-172
- **M.L. ERMINI et al.** *Biosen. and Bioele.,* 2014, vol. 61, 28-37
- **K. CHANG et al.** *Biosen. and Bioele.,* 2015, vol. 66, 297-307
- **BREMNER D et al.** Output-sensitive algorithms for computing nearest-neighbor decision boundaries. *Discrete and Computational Geometry,* 2005, vol. 33 (4), 593604